# EUROPEAN PATENT APPLICATION

(11) **EP 4 759 193 A1**
(43) Date of publication of application: **17.06.2026**
(21) Application number: 25222983.6
(22) Date of filing: 12.12.2025
(51) Int. Cl.: A45D 34/04, A45D 40/26

(54) **A COSMETIC APPLICATOR HEAD**

(30) Priority: 16.12.2024 IT 202400028512
(71) Applicant: Myc Packaging Technology (Suzhou) Co., Ltd, Suzhou, Jiangsu Province 215011 (CN)
(72) Inventor: BOSE, Rahul, New Delhi (IN); Kohli, Manisha, 110048 New Delhi (IN); Srivastava, Smita, 110017 New Delhi (IN)
(74) Representative: Lunati & Mazzoni S.r.L.

(57) **Abstract**

The present invention provides for an applicator head (200) for applying cosmetics or personal care products, defining a main axis (X) and comprising: a main body (210) defining at least a first application surface (212) and comprising: a dispensing channel (102) terminating in a dispensing opening (214), the dispensing channel being extended, at said dispensing opening (214), along a dispensing axis (Y) parallel to said main axis (X), the dispensing channel (102) and the dispensing opening (214) being configured to dispense a product stored in a container; a thermal member (220), made of a thermal material capable of transferring heat to or from the user's skin, constituting at least a second application surface (222) being integrally bonded to the main body (210); the applicator head (200) being characterised in that the first application surface (212) and the second application surface (222) are mainly developed along the main axis (X), and the applicator head (200) comprises a separating surface (213) bordering the first application surface (212) and the second application surface (222) and separating the first application surface (212) and the second application surface (222) so as to keep them separate in use.

## Description

Embodiments of the present disclosure generally relate to an applicator head for applying and dispensing a product, including cosmetic care, or pharmaceutical products, to human skin or keratinous materials, such as hair, eyebrows, face, lips, nails, and/or eyelashes. More specifically, the present disclosure relates to an applicator head that has a thermal member, which provides a thermal effect that creates a cooling sensation upon application.

Cosmetic applicators such as dip or wand applicators are known in the cosmetic industry. Cosmetic packages frequently incorporate these applicators for administering a specific cosmetic or medicinal product contained in the package reservoir. These applicators typically consist of an exterior tubular shell or housing with a reservoir containing a product and an applicator tip positioned at the distal end of the shell or housing. The applicator heads have the form of a brush, spatula, or other applicator structure that is suited for applying a cosmetic or care product such as viscous cosmetics, mascara, eyeliner, lip gloss, hair colour, wound care, skin care, under-eye cosmetics, pharmaceuticals, and like products.

In many cases, these medicinal and cosmetic goods may contain skincare elements such as aloe vera or lanolin, which have a healing or therapeutic effect on injured skin or retain healthy skin. In addition, cosmetic dispensing packages already exist that include a fluid reservoir associated with a rigid and heat-transfer dispenser head for use on the skin, particularly the skin of the face. Furthermore, these products may also incorporate a thermal member that provides a cooling or hot feeling to the skin while the product is applied using the applicator.

The US Patent US11653739B1 assigned to APR Beauty Group Inc describes an applicator head designed to be used with a cosmetic dispensing package to dispense and apply fluid products. The applicator head is partially constructed of a material having thermal capacity, which may hold and transfer heat energy.

Another US patent US11096467B2 assigned to HCT Group Holding Ltd discloses a dispenser having a housing and a reservoir for containing a pharmaceutical or cosmetic care product. An applicator tip is attached to the housing and contains an applicator as well as a product delivery tunnel that runs through the tip and ends at an opening in the applicator. Further, a thermal storage mechanism is located on the applicator, away from the entrance and provides an application surface for contacting a user.

U.S. patent US 9155372 B2, granted to AAxen, Paris FR, and published patent application US 2019/0380472 A1 in the name of HTC Asia Limited, both describe dispensers including a polymeric material portion including the product spill nozzle and a metal portion, bordering and continuous with the polymeric portion. Consequently, the product, when it comes out of the nozzle, is partly on the metal surface and partly on the polymer surface.

Similar devices are also described in patent documents CA 2862466 A1, KR 10-2014-0074573 A, US 11 104 505 B2,
While such applicators are generally satisfactory, there still exists a need for an applicator head that is capable of providing a thermal treatment to the user's skin and is economical to manufacture and is aesthetically pleasing.

In this situation, the technical task underlying the present invention is to devise a cosmetic applicator head that can substantially overcome at least part of the aforementioned drawbacks.

Within said technical task, it is an important purpose of the invention to provide an applicator head both capable of providing a cooling or warming sensation on the user's skin controllable by the user.

Another purpose of the present invention is to provide an applicator head that allows a product to be applied locally or topically to a selected area of a keratin surface and provides a thermal sensation to the keratin surface. The product may include viscous liquid, semisolid or powder products.

Yet another purpose of the present invention is to provide an applicator head that is easy to use, inexpensive to manufacture, and visually appealing.

Further objectives of the invention will appear as the description proceeds.

The specified technical task and purposes are achieved by the applicator head as claimed in the attached claim 1.

Preferred technical solutions are highlighted in dependent claims.

A more complete appreciation of the present disclosure and many of the attendant advantages thereof will be readily obtained as the same becomes better understood by reference to the following detailed description when considered in connection with the accompanying drawings, wherein:
FIG. 1 illustrates a front view of a cosmetic package in a closed position according to a first embodiment of the present disclosure;
FIG. 2 illustrates a front view of an applicator head of the cosmetic package of FIG 1;
FIG. 3 illustrates a top view of the applicator head of FIG 2;
FIG. 4 illustrates a longitudinal cross-sectional view of the applicator head of FIG 2;
FIG. 5 illustrates a side view of the applicator head of FIG 2;
FIG. 6 illustrates a cross-sectional side view of the applicator head of FIG 2;
FIG. 7 illustrates an exploded view of the applicator head of FIG 2;
FIG. 8 illustrates an exploded view of the applicator head of FIG. 2, showing variant implementation according to the first embodiment of the invention;
FIG. 9 illustrates the perspective view of a cosmetic package comprising a cosmetic applicator head, according to a second embodiment of the present disclosure;
FIG. 10 illustrates an exploded view of the cosmetic package comprising the cosmetic applicator head of FIG. 9;
FIG. 11 illustrates a section view of the applicator head of FIG 10;
FIG. 12 illustrates a side view of the applicator head of FIG. 10;
FIG. 13 illustrates a section side view of the applicator head of FIG 12; and
FIG. 14 illustrates an exploded view of the applicator head of FIG 10,
FIG. 15 illustrates an axonometry view of a further example of the applicator head;
FIG. 16 illustrates a front view of the example in FIG 15;
FIG. 17 illustrates a lateral median section of the example in FIG 15,
FIG. 18 illustrates a side view of the example in FIG 15.

As shown throughout the drawings, like reference numerals designate like or corresponding parts. While illustrative embodiments of the present disclosure have been described and illustrated above, it should be understood that these are exemplary embodiments of the disclosure and are not to be considered as limiting. Additions, deletions, substitutions, and other modifications can be made without departing from the spirit or scope of the present disclosure. Accordingly, the present disclosure is not to be considered to be limited by the following description.

Throughout this specification, the terms "comprise," "comprises," "comprising" and the like, shall consistently mean that a collection of objects is not limited to those objects specifically recited.

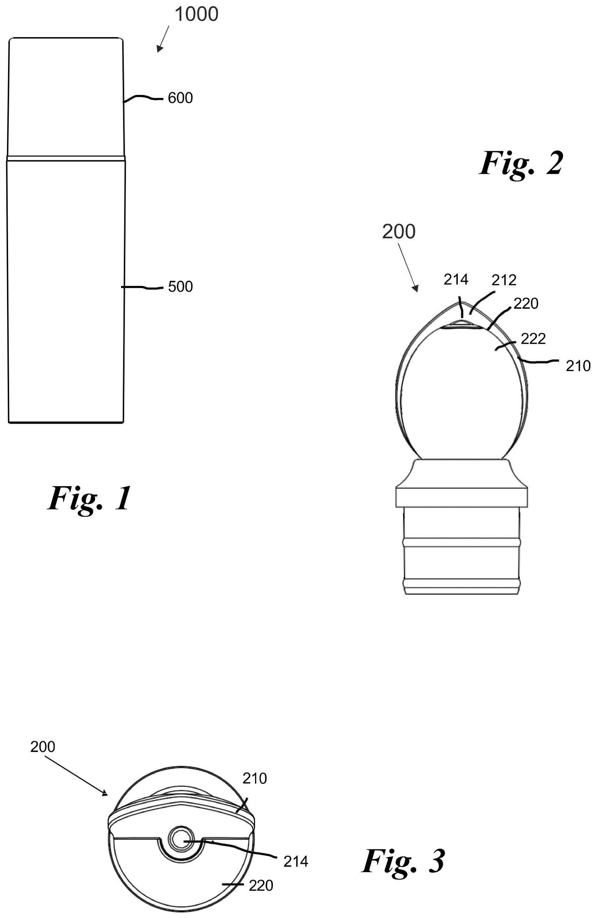

FIGS. 1-8 show an applicator head of the present disclosure according to a first embodiment.

FIG. 1 represents a cosmetic dispensing package **1000** comprising a housing **500** and a cap **600** configured to be coupled to the housing 500 when the cosmetic dispensing package is not in use.

In one of the implementations, the housing 500 may be configured to receive an inner container (not shown) having a reservoir for containing a product including a cosmetic or a care product. The cosmetic or care products include viscous cosmetics, mascara, eyebrow powder, lip gloss, hair color, skincare, under-eye cosmetics, powder, serum, pharmaceutical and like products. It is preferably a creamy product.

FIGGs. 2-8 show the applicator head **200** according to a first embodiment of the present disclosure. The applicator head 200 is used to apply the product including a cosmetic or a care product. The cosmetic or care products include viscous cosmetics, mascara, eyebrow powder, lip gloss, hair color, skincare, under-eye cosmetics, powder, serum, pharmaceutical and like products. Further, the product is to be applied locally or topically to a selected area of a keratin surface and may include viscous liquid, semisolid or powder products.

As shown in FIG. 2, the applicator head 200 includes a main body **210** and a thermal member **220** of the main body 210 and suitable for applying the product.

The main body 210 defines at least a first application surface **212,** part of the outer surface.

The thermal member 220 is preferably coupled to one side of the main body 210. An applicator surface **212** of the main body 210 together with a second application surface **222** of the thermal member 220 is suitable for dispensing and applying the cosmetic and care products.

The applicator head 200 defines a main axis X preferably vertical and/or preferably aligned with the prevailing extension axis of the package for dispensing cosmetics 1000.

The main body 210 further comprises a dispensing channel 102 terminating in a dispensing opening 214 configured to dispense a product stored in a container, as shown in FIGS. 2 to 7. The dispensing channel is preferably extended, at the dispensing opening 214, along a dispensing axis Y parallel to the main axis X.

Further, in some embodiments the dispensing opening can be such as a hole, a slit or an opening. When the product is applied, the user can bring the product through the dispensing opening into contact with his/her skin. The application surfaces 212 and 222 are used for applying the dispensed product. The application surface 222 is also used for providing thermal sensation to the user's skin. The thermal member 220 allows the users to massage a larger area of the keratinous surface.

Application surfaces 212 and 222 are preferably flat or convex.

Also, preferably, the first application surface 212 and the second application surface 222 are mainly developed along the main axis X.

Further still, preferably, the applicator head 200 includes a separating surface **213** bordering the first application surface 212 and the second application surface 222 and defining a rim separating the first application surface 212 and the second application surface 222 so as to keep them separate in use. Therefore, the separation surface 213 is such that if the cosmetic product, in particular a creamy product, is used normally, the product will not transfer between the first and second application surfaces 212, 222 unless the user transfers it on purpose, such as with a finger or with special movements. The separating surface then acts as a rim or a wall between the two application surfaces 212, 222.

Furthermore, advantageously, the separation surface 213 defines at the interface with the first application surface 212 and/or the second application surface 222 an edge **215,** so as to cooperate in keeping the first application surface 212 and the second application surface 222 separate in use. Thus, edge 215 is a discontinuity between application surfaces 212, 222 that promotes separation between them.

Also, preferably, the first application surface 212 and the second application surface 222 are facing different sides. More preferably, so that the perpendicular and outgoing half-lines, essentially at each point, with respect to the application surfaces 212, 222, are divided by a single plane parallel to the main axis X.The said features confer the advantage that the user can select the area of use and the associated thermal sensation. In contrast, in the prior art mentioned above, the thermal sensation is not selectable because the product is spread over the application surfaces and the thermal sensation is given by a mixture of the two surfaces.

Preferably, the separating surface 213 extends predominantly along a line passing through a plane parallel to the dispensing axis Y.

According to an aspect of the present disclosure, the thermal member 220 is made of a thermal material, and preferably different from the material of the main body 210. The thermal member 220 is made of a material with high thermal responsivity or conductivity compared to that of the user's surface to be treated. This thermal material has preferably a thermal conductivity coefficient (λ) greater than 10 W/(m*K). The thermal responsivity of the thermal member 220 is higher than that of the main body 210, the thermal member 220 retains thermal charge which is capable of being released during application. More particularly, the thermal member 220 is made of a material capable of holding and retaining a thermal charge. In one implementation, the thermal member 220 is made of metal. In other cases, any acceptable material capable of storing heat or cold during cosmetic or care product application can be used. Other possible materials include but are not limited to, metals (e.g., aluminium, titanium, steel, nickel, tin, copper, brass, alloys thereof), steel, ceramics, stones, gemstones, high-density polymers, composites, and the like.

The main body 210 is prefarbly made of a thermoplastic polymer, for example, which is non-reactive with the cosmetic or care product stored in the container. Preferably, the main body 210 is made of plastic, polyethylene, polypropylene, ABS, silicon, plastic, glass or any other suitable material.

FIG. 3 is a more detailed top view of the applicator head 200. Again, the applicator head 200 generally comprises the main body 210 together with the thermal member 220, and the dispensing opening 214 configured to dispense a product stored in the container.

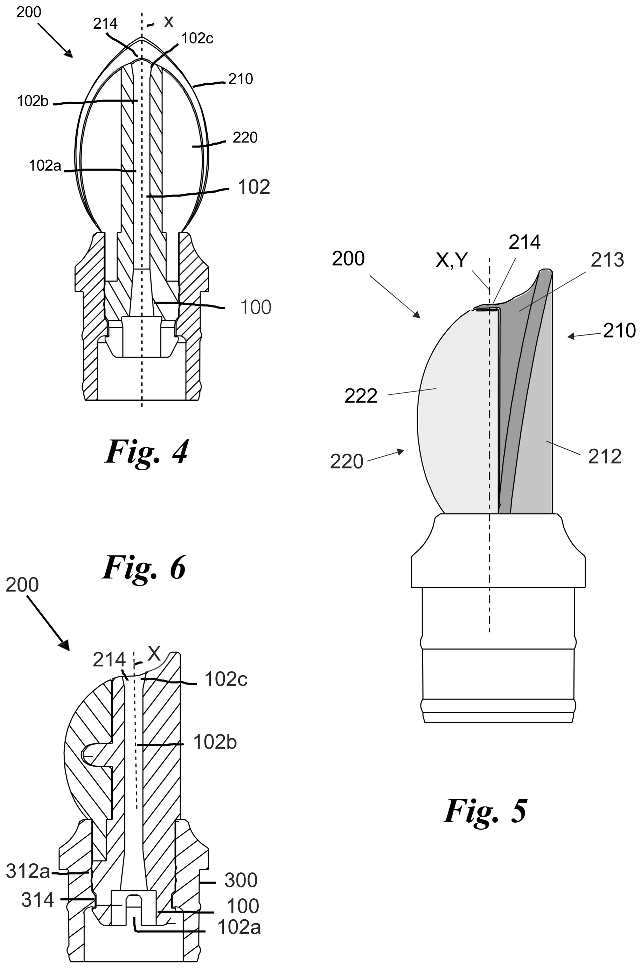

Referring to FIGS. 5 and FIG. 6, the thermal member 220 is convex in a longitudinal direction. The applicator surface 222 of the thermal member is curvilinear or curving in an ever-changing slope to allow for easy and even distribution of the product on the skin of a user. In some implementations, the applicator surface 222 may have any other suitable shape and size, or surface finish desired for a given application. According to an aspect of the present disclosure, a longitudinal axis **A** of the thermal member 220 is aligned with a main axis X of the main body 210, as shown in FIG. 4, FIG. 5, and FIG. 6. The applicator surface 212 of the main body 210 and the applicator surface 222 of the thermal member 220 are preferably mainly distinguished along a line passing through a plane parallel to the dispensing axis Y and thus preferably lie in different vertical planes and do not form the second preferably smooth and continuous application surface.

Again FIG. 4 and FIG. 6 illustrate the cross-sectional view of the applicator head 200, including the main body 210 extending longitudinally along a longitudinal axis X. The main body 210 comprises a connecting member 100 configured to receive the product stored in the container. The main body 210 and the connecting member 100 form a channel **102** that is configured to receive the product stored in the container. The channel 102 also enables the product to pass from the container to a dispensing opening 214 onto the application surfaces 222 and 212. In an aspect of the present disclosure, the main body 210 and the connecting member 100 may be integrally formed of the same material or as distinct members linked using any acceptable fixing techniques known in the art. For example, the main body 210 is fastened to the connecting member 100 using adhesive, heat-sealing, force-fitting, snap-fit, crimping, or screw-fastening. In some implementations, the main body 210 is made integrally with the connecting member 100, either from the same material or from different materials, e.g. dual-injection moulding.

Referring to FIG. 6, the channel 102 comprises a first channel portion **102a** formed in the connecting member 100, a second channel portion **102b** formed in the main body 210, and a third channel portion **102c** formed in the main body 210, wherein the third channel portion 102c is flared and further terminating at the dispensing opening 214 and defining the dispensing axis of the dispensing opening 214. Further, the longitudinal axis of the thermal member, the main body, the connecting member and the dispensing axis of the dispensing opening are aligned, as shown in FIG. 4 and FIG. 6.

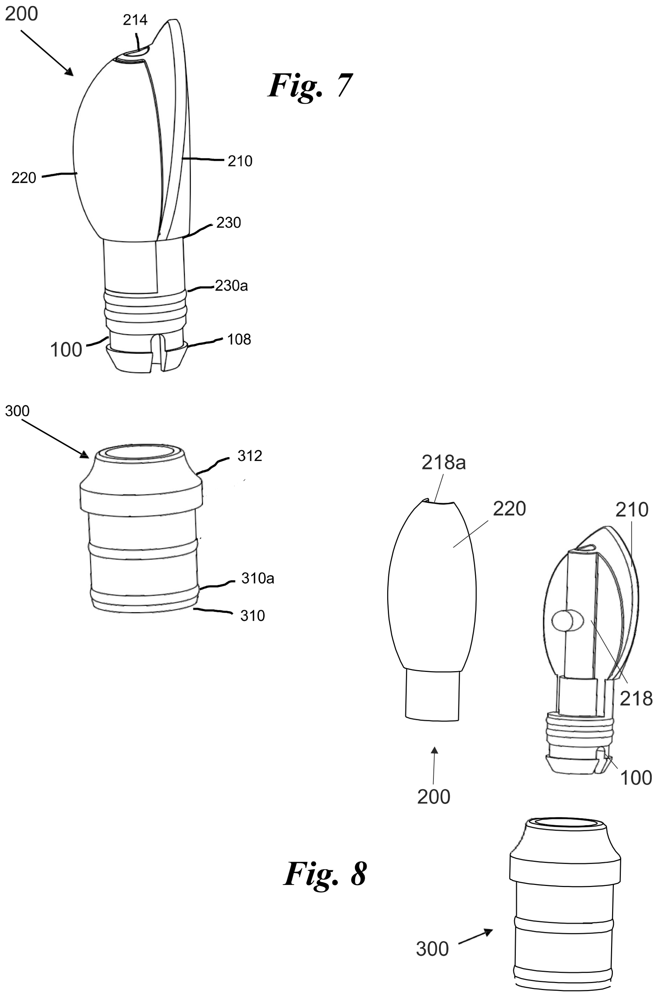

Referring to FIG. 7 and FIG. 8, a collar head 300 is configured to be connected to the applicator head 200 at one end and to the container (not shown) at another end such that the thermal member 220, the main body 210 and the connecting member 100 are coaxial with a longitudinal axis of the collar head 300.

According to an aspect of the present disclosure, the collar head 300 may further comprise a first engagement member **310a** provided at a lower portion **310** of the collar head 300, the first engagement members 310a configured to mate with complementary engagement members provided in the container (not shown) inside the housing 500, to fasten the collar head 300 to the container.

Again as shown in FIG. 6 and FIG. 7, the collar head 300 further comprises second engagement members **312a** provided at an upper portion **312** of the collar head 300, the second engagement members 312a configured to couple with complementary engagement members **230a** provided in a lower portion **230** of the main body 210, to fasten the main body 210 to the collar head 300. The collar head 300 further comprises third engagement member 314a configured to couple with complementary engagement member 108 of the connecting member 100, to fasten the connecting member 100 to the collar head 300.

In one implementation, the third engagement member **314a** on the collar head 300 has protrusions configured to couple with the complementary groove **108** of the connecting member 100. In some implementations, the fastening can be force-fitting, snap-fastening, crimping, screw-fastening, threaded attachment, press or friction fit, one or more hinges, or any other suitable means of attachment.

Referring to FIG. 8, the main body 210 comprises preferably a coupling feature 218 with a longitudinal axis substantially parallel with the longitudinal axis X of the main body 210. The coupling feature 218 is configured to couple the thermal member 220 such that the longitudinal axis A of the thermal member 220 is aligned with a longitudinal axis X of the main body 210, the longitudinal axis of the connecting member 100 and the dispensing axis of the dispensing opening 214.

In the exemplary embodiment, the thermal member 220 includes a coupling groove 218a to couple with the coupling feature 218 in the main body 210. Further, the thermal member 220 may be secured to the main body 210 by a press-fit, snap-fit, adhesive, and/or engagement by one or more engagement features or by any other means of engagement known in the field. In any case, preferably, the main body 210 is integrally constrained to the thermal member 220.

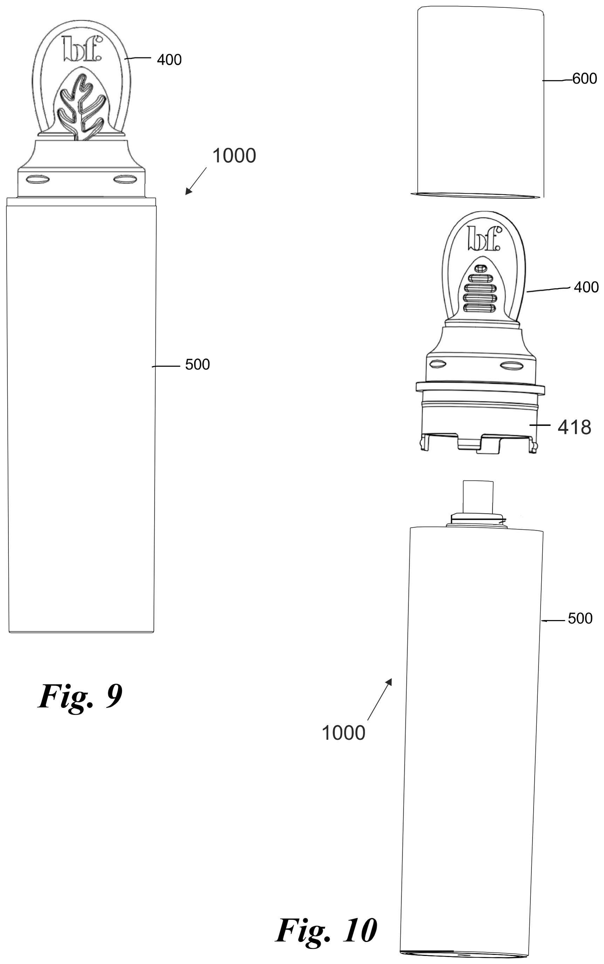

FIGS. 9-14 show a variant of the applicator head of the present disclosure according to a second embodiment.

FIG. 9 and FIG. 10 represent a cosmetic dispensing package 1000 comprising a housing 500 and a cap 600 configured to be coupled to the housing 500 and a cosmetic applicator head 400 placed on the housing 500. The applicator head **400** and the container (not shown) for holding cosmetic or care product housing 500 is coupled using a collar head **418** using snap-fit, j-lock, magnetic engagement, screw threads, et cetera as shown in Fig. 10.

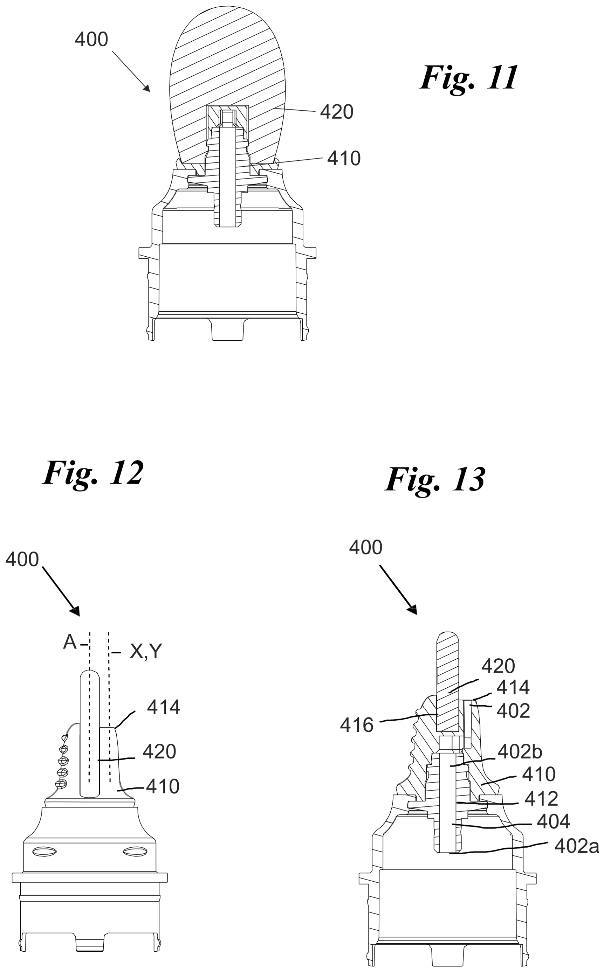

FIGS. 11-14 show the applicator head 400 according to the second embodiment of the present disclosure. The applicator head 400 may be used to apply the product including a cosmetic or a care product. The cosmetic or care products include viscous cosmetics, mascara, eyebrow powder, lip gloss, hair color, skincare, under-eye cosmetics, powder, serum, pharmaceutical and like products. Further, the product is to be applied locally or topically to a selected area of a keratin surface and may include viscous liquid, semisolid or powder products.

As shown in FIG. 11, FIG. 12 and FIG. 13, the applicator head 400 includes a main body **410** and a thermal member **420.** The thermal member 420 is coupled to the main body 410. The main body 410 further comprises a dispensing opening **414** configured to dispense a product stored in a container, as shown in FIG. 12 and FIG. 13. Further, in some embodiments the dispensing opening can be such as a hole, a slit or an opening. When the product is applied, the user can bring the product through the dispensing opening into contact with his/her skin.

According to an aspect of the present disclosure, the thermal member 420 is made of a material different from the material of the main body 410. The thermal member 420 is made of a material with high thermal responsivity compared to that of the user's surface to be treated. The thermal responsivity of the thermal member 420 is higher than that of the main body 410, the thermal member 420 retains the thermal charge which is capable of being released during application. More particularly, the thermal member 420 is made of a material capable of holding and retaining a thermal charge. In one implementation, the thermal member 420 can be made of metal. In other cases, any acceptable material capable of storing heat or cold during cosmetic or care product application can be used. Other possible materials include but are not limited to, metals (e.g., aluminium, titanium, steel, nickel, tin, copper, brass, alloys thereof), steel, ceramics, stones, gemstones, high-density polymers, composites, and the like.

The main body 410 is made of plastic, for example, which is non-reactive with the cosmetic or care product stored in the container. Preferably, the main body 410 is made of plastic, polyethylene, polypropylene, ABS, silicon, plastic, glass or any other suitable material.

FIG.12 and Fig.13 illustrate the side view and the cross-sectional view of the applicator head 400 respectively, including the main body 410 extending longitudinally along a longitudinal axis X. The main body 410 comprises a connecting member **412** configured to receive the product stored in the container. The main body 410 and the connecting member 412 form a channel **404** that is configured to receive the product stored in the container. The channel 404 also enables the product to pass from the container to a dispensing opening 414 defining the dispensing axis Y of the dispensing opening 414.

The channel 404 comprises a first channel portion **402a** formed in the connecting member 412, a second channel portion **402b** formed in the main body 410, and a third channel portion **402c** formed in the main body 410. The first channel portion 404a is coaxial with the longitudinal axis X of the main body 410, the second channel portion 402b is disposed at a first predetermined angle with respect to the first channel portion 402a, and the third channel portion 402c may disposed at a second predetermined angle with respect to the second channel portion 402b. In present embodiment, the third channel portion 402c is parallel to the first channel portion 402a with a dispensing axis of the third channel portion 402c offset from the longitudinal axis X of the main body 410. Further, the third channel portion 402c is terminating at the dispensing opening 414. The dispensing axis of the third channel portion 402c is parallel to the longitudinal axis X of the main body 410.

In an aspect of the present disclosure, the main body 410 and the connecting member 412 may be integrally formed of the same material or as distinct members linked using any acceptable fixing techniques known in the art. For example, the main body 410 can be fastened to the connecting member 412 using adhesive, heat-sealing, force-fitting, snap-fit, crimping, or screw-fastening. In a different implementation of the present disclosure, the main body 410 is made integrally with the connecting member 412, either from the same material or from different materials, e.g. dual-injection moulding.

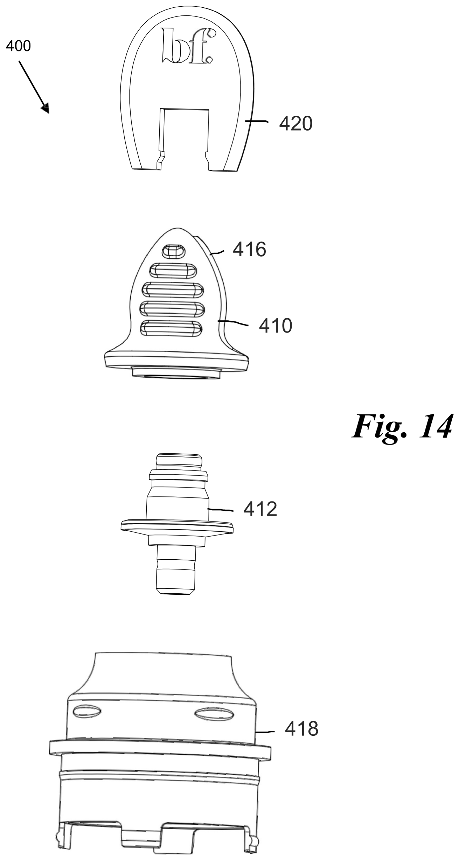

According to the second embodiment of the present disclosure, the main body 410 comprises a coupling feature **416** with a longitudinal axis substantially along the longitudinal axis X of the main body 410, as shown in FIGS 13 and 14. The coupling feature 416 is configured to couple the thermal member 420 such that the longitudinal axis A of the thermal member 420 is substantially aligned with the longitudinal axis X of the main body 410 and the longitudinal axis of the connecting member 412; and the longitudinal axis A of the thermal member 420 is parallel to the dispensing axis Y of the dispensing opening 414, as shown in FIGS 12 and 13. The thermal member 420 is be secured to the main body 410 by a press-fit, snap-fit, adhesive, and/or engagement by one or more engagement features or by any other means of engagement known in the art.

Referring to FIGS. 13 and 14 the main body 410 includes the coupling feature as a coupling slot 416 to receive and couple the thermal member 420 with the main body 410. Additionally, coupling slot 416 primarily secures precisely employing snap or interference/fitment with the respective thermal member. Additionally, the coupling slot 416a can be constructed of any diameter and depth corresponding to the dimensions of the thermal member 420.

Referring to FIG. 14, an exploded view of the cosmetic package is shown where a collar head 418 is configured to be connected to the applicator head 400 at one end and to the container (not shown) in the housing 500 at another end.

In one implementation, the collar head 418 is snap fitted with the connecting member 412. In other implementations of the present disclosure, the collar head **418** and the connecting member 412 can be coupled using force-fitting, crimping, screw-fastening, threaded attachment, press or friction fit, one or more hinges, or any other suitable means of attachment.

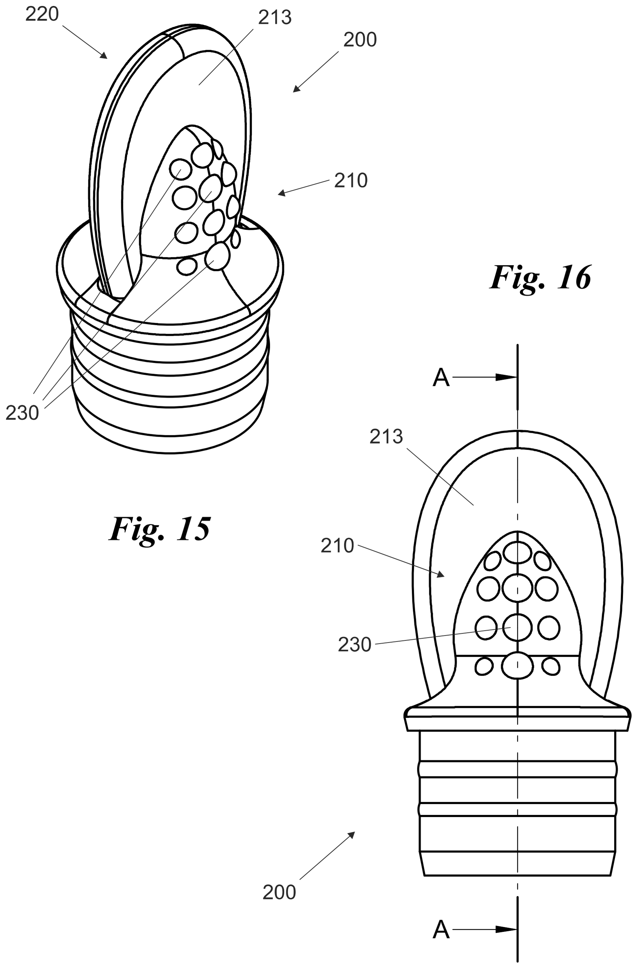

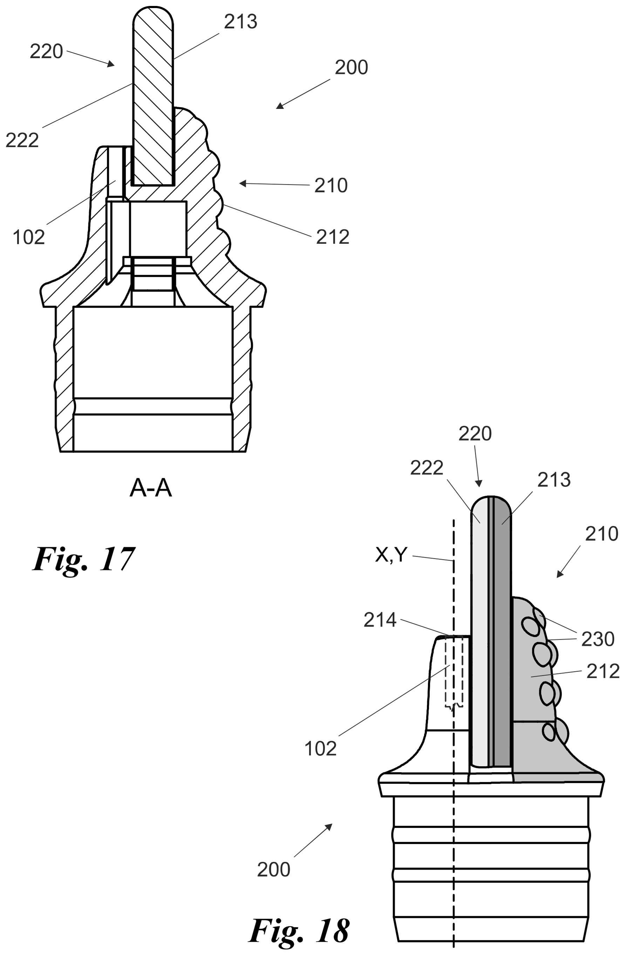

Figures 15-18 show a further example of an applicator head according to the invention. a first application surface 212 defined by a convex or flat main extension surface and protrusions 230 protruding from the convex or flat main extension surface. Alternatively, the second application surface 222 could have the same conformation.

Although the present disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure and the scope thereof is determined by the claims that follow.

## Claims

1. An applicator head (200) for applying cosmetics or personal care products, defining a main axis (X) and comprising:
- a main body (210) defining at least a first application surface (212) and comprising:
- a dispensing channel (102) terminating in a dispensing opening (214),
- the dispensing channel being extended, at said dispensing opening (214), along a dispensing axis (Y) parallel to said main axis (X),
- the dispensing channel (102) and the dispensing opening (214) being configured to dispense a product stored in a container;
- a thermal member (220),
- made of a thermal material capable of transferring heat to or from the user's skin,
- constituting at least a second application surface (222),
- being integrally bonded to the main body (210);
- the applicator head (200) being **characterised in that:**
- the first application surface (212) and the second application surface (222) develop predominantly along the main axis (X),- and from the fact that the applicator head (200) includes a separation surface (213) bordering the first application surface (212) and the second application surface (222) and defining a separating rim between the first application surface (212) and the second application surface (222) such that the cosmetic product used normally, does not transfer between the first and second application surface (212, 222) unless the user purposely transfers it.

2. Applicator head according to claim 1, wherein the separation surface (213) defines at the interface with the first application surface (212) and/or the second application surface (222) an edge (215), so as to keep the first application surface (212) and the second application surface (222) separate in use.

3. Applicator head according to any previous claim, whereinthe first application surface (212) and the second application surface (222) face each other from different sides, so that the perpendicular and outgoing half-lines at each point from the application surfaces (212, 222) are divided by a single plane parallel to the main axis (X),

4. Applicator head according to any of the previous claims, wherein the separation surface (213) extends predominantly along a line passing through a plane parallel to the dispensing axis (Y).

5. Applicator head according to any of the previous claims, wherein said separating surface (213) is part of the surface of said main body (210).

6. Applicator head according to any previous claim, wherein the thermal member (220) and the main body (210) are mutually constrained by means of a coupling element (218) having a longitudinal axis substantially parallel to the longitudinal axis of the main body (210).

7. Applicator head according to any previous claim, wherein the first application surface (212) and the second application surface (222) are convex or plane.

8. Applicator head according to any previous claim, wherein the first application surface (212) and/or the second application surface (222) are defined by a convex or plane main extension surface and protuberances (230), protruding from said convex or plane main extension surface.

9. Applicator head according to any previous claim, wherein the main axis (X) is vertical.

10. Cosmetic container comprising an applicator head according to any previous claim, wherein said cosmetic container extends mainly along said main axis (X).
